# EUROPÄISCHE PATENTSCHRIFT

(11) **EP 3 749 805 B1**
(45) Veröffentlichungstag und Bekanntmachung des Hinweises auf die Patenterteilung: **20.08.2025**
(21) Anmeldenummer: 19704566.9
(22) Anmeldetag: 01.02.2019
(51) Int. Cl.: D21H 27/10, D21H 27/00, D21H 19/12, D21H 19/14

(54) **VERPACKUNGSMATERIAL, VERFAHREN ZUR HERSTELLUNG DES VERPACKUNGSMATERIALS UND DESSEN VERWENDUNG**
PACKAGING MATERIAL, METHOD FOR PRODUCING SAID PACKAGING MATERIAL, AND USE THEREOF
MATÉRIAU D'EMBALLAGE, PROCÉDÉ DE FABRICATION DUDIT MATÉRIAU D'EMBALLAGE ET SON UTILISATION

(30) Priorität: 05.02.2018 DE 102018102508
(43) Veröffentlichungstag der Anmeldung: 16.12.2020
(73) Patentinhaber: Huhtamaki Flexible Packaging Germany GmbH & Co. KG, 87671 Ronsberg (DE)
(72) Erfinder: OBERMAYER, Johannes, 87760 Lachen (DE); WALTERS, Nils, 15040 Pietra Marazzi (AL) (IT); GMEINDNER, Andreas, 87634 Obergünzburg (DE); SCHAEFTNER, Markus, 87634 Obergünzburg (DE)
(74) Vertreter: Meissner Bolte Partnerschaft mbB
(86) Internationale Anmeldenummer: PCT/EP2019/052489
(87) Internationale Veröffentlichungsnummer: WO 2019/149882

(56) Entgegenhaltungen:
- EP-A1- 2 586 606
- WO-A1-2010/113885
- WO-A1-2012/044229
- DE-A1- 10 322 267
- DE-T2- 69 322 269
- US-A- 3 503 782
- US-A- 4 434 259
- US-A1- 2002 110 675

## Beschreibung

Die Erfindung betrifft ein Verpackungsmaterial gemäß dem Oberbegriff von Patentanspruch 1, ein Verfahren zum Herstellen eines solchen Verpackungsmaterials gemäß dem Oberbegriff von Patentanspruch 9 und die Verwendung eines solchen Verpackungsmaterials gemäß dem Oberbegriff von Patentanspruch 10.

Derartige Verpackungsmaterialien werden üblicherweise als sogenannte "Medical Papers" benötigt und verwendet und dienen zum Herstellen und/oder zum Verschließen von, insbesondere medizinischen, Behältern und Verpackungen.

Solche Verpackungsmaterialien, nämlich "Medical Papers", basieren üblicherweise auf einem Verpackungsgrundmaterial auf Papierbasis, das so beschichtet ist, dass es mit einem Gegenpart verbunden, nämlich insbesondere verklebt oder versiegelt werden kann. Hierbei ist es zum einen wichtig, dass das Verpackungsmaterial, respektive das "Medical Paper", zum Öffnen der Verpackung sauber von dem Gegenpart abgelöst werden kann, ohne dass das Verpackungsgrundmaterial bei diesem Öffnungsvorgang zerreißt. Darüber hinaus muss nach einem Öffnen der Verpackung sichergestellt sein, dass sich die Verpackung mit dem "Medical Paper" nicht wieder verschließen läßt, so dass eindeutig erkennbar ist, dass eine einmal geöffnete Verpackung bereits geöffnet wurde.

Gemäß dem bisherigen Stand der Technik wurden derartige Verpackungsmaterialien dadurch erzeugt, dass das Verpackungsgrundmaterial auf Papierbasis mit Schmelzklebstoff beschichtet wurde, der entweder in Form einer Schmelzklebstoff-Lösung in Toluol oder direkt auf das Papier aufgebracht wurde.

Dieses Verfahren sowie die damit hergestellten Verpackungsmaterialien weisen jedoch erhebliche Nachteile auf, die bei einem lösemittelbasierten Auftrag des Schmelzklebstoffs auf das Papier darin bestehen, dass in dem Verpackungsmaterial ein hoher Restlösemittelanteil verbleibt und das Endprodukt somit Toluol aufweist.

Darüber hinaus ist bei einem solchen lösemittelbasierten Schmelzklebstoffauftrag das Auftragsgewicht des Schmelzklebstoffs sehr hoch und liegt im Bereich von 6 g/m² bis 11 g/m² und höher. Dieses hohe Auftragsgewicht des Schmelzklebstoffs führt zum einen zu einem sehr hohen Endgewicht des Endprodukts sowie aufgrund der dicken Schmelzklebstoffschicht zu einer Verschärfung des Restlösemittelproblems, da der Schmelzklebstoff eine Senke für das Lösemittel Toluol darstellt.

Demgegenüber bringt ein direkter Schmelzklebstoffauftrag den Nachteil mit sich, dass die Maschinengeschwindigkeit bei der Herstellung des Verpackungsmaterials gering ist, so dass die Produktivität der Anlage sinkt. Darüber hinaus führt ein dicker Schmelzklebstoffauftrag ferner zu einem schlechten Hottack, d.h. zu einer schlechten initialen Siegelfestigkeit beim Herstellen und/oder Verschließen des Behälters oder der Verpackung.

Diese Nachteile wirken sich ungünstig auf die Qualität bisheriger Verpackungsmaterialien aus, die als "Medical Paper" genutzt werden.

Der Erfindung liegt die Aufgabe zugrunde unter Vermeidung oben genannter Nachteile ein Verpackungsmaterial zur Verfügung zu stellen, das als "Medical Paper" geeignet ist, einfach und kostengünstig herstellbar ist und neben einer Sterilisierbarkeit eines Produkts, das in einer mit dem Verpackungsmaterial hergestellten oder verschlossenen Verpackung enthalten ist, ferner eine saubere Ablösbarkeit des Verpackungsmaterials von einem Gegenpart der Verpackung gewährleistet, ohne dass die Verpackung mit dem Verpackungsmaterial wieder verschlossen werden könnte. Des Weiteren umfasst die Aufgabe der Erfindung ferner, die Herstellung eines solchen Verpackungsmaterials sowie dessen Verwendung zur Verfügung zu stellen.

Diese Aufgabe wird durch ein Verpackungsgrundmaterial gemäß Patentanspruch 1, durch ein Verfahren zum Herstellen eines solchen Verpackungsmaterials gemäß dem Patentanspruch 9 und durch die Verwendung eines solchen Verpackungsmaterials gemäß Patentanspruch 10 gelöst.

Insbesondere wird die Aufgabe durch ein Verpackungsmaterial aus Papier oder mit einer Papieraußenseite gelöst, wobei die Papieraußenseite, insbesondere vollflächig, mit einem Trennlack und der Trennlack,musterförmig, nämlich gitterförmig, mit einem Siegelmedium beschichtet ist.

Ein wesentlicher Punkt der Erfindung liegt darin, dass erfindungsgemäß ein Trennlack, insbesondere vollflächig, auf die Außenseite des Verpackungsgrundmaterials aus Papier aufgetragen wird. Der Trennlack gewährleistet, dass die Beschichtung des Verpackungsgrundmaterials, die aus Trennlack und Siegelmedium besteht, gemäß einer ersten Variante vollständig von dem Verpackungsgrundmaterial, d.h. von dem Papier, abgelöst werden kann, ohne dass es zu einem Papierfaserriss kommt. Bei einem Öffnen der Verpackung verbleibt somit die ursprünglich an dem Verpackungsgrundmaterial angeordnete Beschichtung aus Siegelmedium und Trennlack an dem Gegenpart, an welchem das erfindungsgemäße Verpackungsmaterial aufgebracht wurde, so dass das erfindungsgemäße Verpackungsmaterial bei einem Ablösen von der Verpackung, respektive bei einem Öffnen der Verpackung, quasi delaminiert und dadurch zerstört wird, so dass ein Wiederverschluss der Verpackung keinesfalls möglich und somit sicher vermieden wird.

Der Trennlack kann erfindungsgemäß nur in den zu siegelnden Bereichen des erfindungsgemäßen Verpackungsmaterials oder aber nach Wunsch auch vollflächig an dem Verpackungsgrundmaterial angeordnet sein.

Das Siegelmedium seinerseits ist mit einem Muster, d.h. gitterförmig, auf dem Trennlack angeordnet. Das Siegelmedium kann hierbei ebenfalls auf die zu siegelnden Bereiche des erfindungsgemäßen Verpackungsmaterials beschränkt aufgebracht sein.

Gemäß einer zweiten erfindungsgemäßen Variante, durch die ebenfalls gewährleistet ist, dass das erfindungsgemäße Verpackungsmaterial bei einem Ablösen von der Verpackung, respektive bei einem Öffnen der Verpackung, strukturell verändert und dadurch zerstört wird, so dass ein Wiederverschluss der Verpackung keinesfalls mehr möglich und somit erfindungsgemäß sicher vermieden wird, verbleibt ein Teil des Trennlacks an dem Verpackungsgrundmaterial. Hierbei wird der Trennlack bei einem Öffnen der Verpackung beispielsweise gebrochen und verbleibt im Wesentlichen an den Stellen an dem Verpackungsgrundmaterial, an welchen der Trennlack bei einem intakten Verpackungsmaterial nicht mit Siegelmedium beschichtet ist. Der Trennlack wird bei dieser Variante somit nicht vollständig von dem Verpackungsgrundmaterial getrennt; das Siegelmedium verbleibt jedoch zur Gänze an dem Gegenpart, mit welchem das Siegelmedium beim Bilden und/oder Schließen des Behälters bzw. der Verpackung versiegelt wurde.

Ein wesentlicher Vorteil eines musterförmigen Auftrags des Siegelmediums besteht ferner darin, dass das erfindungsgemäße Verpackungsmaterial bei einem nicht vollflächigen Auftrag des Siegelmediums eine hohe Gasdurchlässigkeit behält, so dass eine Sterilisierbarkeit des verpackten Produkts mit Ethylenoxid auch bei einer mit dem erfindungsgemäßen Verpackungsmaterial hergestellten und/oder verschlossenen Verpackung problemlos möglich ist.

Gemäß der Erfindung ist der erfindungsgemäße Trennlack ein Trennlack auf Polyamidbasis und gewährleistet bei einem Öffnen der Verpackung einerseits ein problemloses Ablösen von dem Verpackungsgrundmaterial und gewährleistet darüber hinaus aber auch eine hinreichende Adhäsion zwischen dem Trennlack und dem Siegelmedium.

Das Siegelmedium ist erfindungsgemäß aus einer Gruppe ausgewählt, die gegen Polyolefin(e), insbesondere Polyethylen oder Polypropylen, und/oder gegen Polyester, insbesondere Polyethylenterephtalat, und/oder gegen Papier und/oder gegen Polystyrol oder Mischungen vorgenannter Substanzen siegelt und Polyolefin(e), insbesondere Polyethylen oder Polypropylen, Polyester, insbesondere Polyethylenterephtalat, oder Mischungen vorgenannter Substanzen sowie Ethylen-Vinylacetat-Copolymer umfasst. Bei diesen Siegelmedien handelt es sich um gängige und kostengünstige heißsiegelfähige Materialien, die gegen diverse Substrate siegelbar sind, die beispielsweise aus Polyethylen, Polypropylen, Polyethylenterephtalat, Papier oder Polystyrol hergestellt sind oder Siegelflächen mit oder aus diesen Materialien aufweisen. In vorteilhafter Weise können mit den genannten Siegelmaterialien somit optimale Siegelnahtfestigkeiten erreicht werden, die im Bereich von 1,0 N/15mm bis 2,0 N/15mm liegen.

Gemäß einer Ausführungsform der Erfindung ist das Siegelmedium erfindungsgemäß in Form einer Dispersion auf den Trennlack aufgebracht, die aus einer Mischung von Wasser und Ethylen-Vinylacetat-Copolymer sowie gegebenenfalls einem Anteil eines Alkohols, insbesondere Ethanol, Propanol, Isopropanol oder Butanol, im Bereich von 0,01 Gew.-% bis 8 Gew.-%, bevorzugt im Bereich von 0,1 Gew.-% bis 6,5 Gew.-% und besonders bevorzugt im Bereich von 3,5 Gew.-% bis 5 Gew.-% und/oder gegebenenfalls einem weiteren Siegelmedium besteht.

Gemäß einer erfindungsgemäßen Alternative umfasst die Dispersion außer Wasser und Ethylen-Vinylacetat-Copolymer kein weiteres, insbesondere kein weiteres organisches, Lösungsmittel.

Gemäß einer bevorzugten Ausführungsform der Erfindung ist diese Dispersion eine Heißsiegeldispersion.

Erfindungsgemäß ist das Siegelmedium mit einem Auftragsgewicht im Bereich von 0,5 g/m² bis 6 g/m², vorzugsweise im Bereich von 1,5 g/m² bis 5 g/m² und besonders bevorzugt im Bereich von 2 g/m² bis 4 g/m² auf den Trennlack aufgebracht. Ein derartiges Auftragsgewicht ermöglicht die Herstellung eines leichten Verpackungsmaterials und damit einhergehend die Herstellung einer leichten Verpackung. Gleichzeitig gewährleistet ein solches Auftragsgewicht und die damit einhergehende Menge an Siegelmedium eine optimale Versiegelung mit dem jeweiligen Gegenpart des erfindungsgemäßen Verpackungsmaterials und zwar sowohl im Hinblick auf eine Dichtigkeit der Verpackung als auch im Hinblick auf eine Siegelfestigkeit der Siegelnaht bereits in heißem Zustand der Siegelnaht und somit einen optimalen Hottack.

Wie bereits vorerwähnt, ist das erfindungsgemäße Verpackungsmaterial gasdurchlässig, so dass eine Ethylenoxid-Sterilisation des in einer mit dem Verpackungsmaterial hergestellten und/oder verschlossenen Verpackung enthaltenen Produkts problemlos möglich ist. Die Gasdurchlässigkeit des erfindungsgemäßen Verpackungsmaterials wird im Wesentlichen dadurch gewährleistet, dass das Verpackungsgrundmaterial des erfindungsgemäßen Verpackungsmaterials aus Papier besteht und/oder zumindest eine dem Trennlack zugewandte Papierschicht aufweist, die aus Fasern besteht, deren Geflecht gasdurchlässig ist. Das zur Herstellung des Verpackungsmaterials verwendete Papiermaterial ist erfindungsgemäß Plasma behandelt, wobei hierbei in vorteilhafter Weise ein Ausfasern des Papiers vermieden wird.

Der Trennlack ist erfindungsgemäß auf der Papierschicht in gasdurchlässiger Schichtdicke aufgebracht.

Im Übrigen stellt das erfindungsgemäße Verpackungsmaterial ein medizinisches Papier dar.

Des Weiteren wird die erfindungsgemäße Aufgabe auch durch ein Verfahren zum Herstellen eines Verpackungsmaterials gemäß vorstehenden Ausführungen gelöst, wobei die folgenden Schritte durchgeführt werden:
- zur Verfügungstellen eines Verpackungsgrundmaterials aus Papier oder mit einer Papieraußenseite;
- insbesondere vollflächiges Beschichten der Papieraußenseite mit einem Trennlack; und
- zumindest abschnittsweises, gitterförmiges, Aufbringen eines Siegelmediums auf den Trennlack.

Im Übrigen wird die erfindungsgemäße Aufgabe auch durch eine Verwendung eines Verpackungsmaterials mit vorstehenden Eigenschaften zum Herstellen eines, insbesondere medizinischen, Behälters gelöst.

Zusammenfassend kann der Gegenstand der Erfindung somit wie folgt beschrieben werden:
Das erfindungsgemäße Verpackungsmaterial ist im Bereich von und/oder als "Medical Paper" anwendbar und erfüllt alle an ein "Medical Paper" gestellten Anforderungen. Das erfindungsgemäße Verpackungsmaterial umfasst eine sterilisierfähige und heißsiegelbare Beschichtung auf Papier und ist gegen diverse Substrate, wie beispielsweise vorwiegend Polyethylen, aber auch gegen Polypropylen und/oder Polyethylenterephtalat sowie gegen Papier und Polystyrol sowie nach Wunsch auch gegen Mischungen der vorgenannten Polyolefine und/oder anderen Materialien siegelbar. Das erfindungsgemäße Verpackungsmaterial ermöglicht einen sauberen Peel ohne Papierfaserriss, wobei die Beschichtung des Verpackungsgrundmaterials vollständig auf die Gegenseite, d.h. den zu siegelnden Gegenpart, übergeht, so dass die Verpackung nicht wieder verschlossen werden kann bzw. eine Öffnung der Verpackung direkt erkennbar ist. Ein Wiederversiegeln einer einmal mit dem erfindungsgemäßen Verpackungsmaterial verschlossenen und dann geöffneten Verpackung ist nicht möglich. Die mit dem erfindungsgemäßen Verpackungsmaterial erzielbare Siegelnahtfestigkeit liegt im Bereich von 1,0 N/15mm bis 2,0 N/15mm.

Da die erfindungsgemäß verwendete Dispersion, insbesondere Heißsiegeldispersion, außer gegebenenfalls Alkohol, kein Lösungsmittel, insbesondere kein anderes bzw. weiteres organisches Lösungsmittel enthält, ist bei dem erfindungsgemäßen Verpackungsmaterial ein Vorhandensein eines solchen anderen organischen Lösungsmittelrückstandes, wie dies beim Stand der Technik zu befürchten war, ausgeschlossen.

In vorteilhafter Weise lässt sich die aus Trennlack und/oder Siegelmedium bestehende Beschichtung des erfindungsgemäßen Verpackungsmaterials mittels Tiefdruck auf das Verpackungsgrundmaterial aufbringen, was in vorteilhafter Weise nicht nur sehr schnell mit einer hohen Maschinengeschwindigkeit, sondern auch sehr präzise und unter Erzeugung eines musterförmigen Auftrags durchführbar ist.

Das erfindungsgemäße Verpackungsmaterial umfasst somit einen, insbesondere vollflächigen, Auftrag eines polyamidbasierten Trennlacks, der eine Ethylenoxid-Sterilisation nicht verhindert und ein sauberes Ablösen der Heißsiegelbeschichtung von einem jeweiligen Gegenpart gewährleistet.

Die Heißsiegelbeschichtung zeichnet sich dadurch aus, dass sie einen guten Hottack und ein breites Verarbeitungsfenster auf einer Abpackmaschine hat und gegen eine Vielzahl von Substraten, wie beispielsweise Polyethylen, Polypropylen, Polyethylenterephtalat, Polystyrol oder Papier siegelfähig ist. Zudem wird aufgrund der Verwendung einer Dispersion die Gefahr eines erhöhten Restlösemittelanteils äußerst gering gehalten oder, je nach verwendeter Dispersion ganz vermieden. Die Heißsiegeldispersion wird bevorzugt als Gitter aufgetragen, wodurch eine Sterilisierfähigkeit einer mit dem erfindungsgemäßen Verpackungsmaterial hergestellten Verpackung mit Ethylenoxid gewährleistet ist.

Die Kombination aus vollflächig aufgetragenem Trennlack und im Gitter aufgetragenen Heißsiegeldispersion bietet zugleich einen sauberen Transfer von Trennlack und Siegelmedium vom Papier, ohne einen Faserriss des Papiers zur Gegenbahn befürchten zu müssen. Ferner gewährleisten der vollflächig aufgetragene Trennlack und die mit Gittermuster aufgetragene Heißsiegeldispersion eine hinreichende Adhäsion zwischen Trennlack und Heißsiegeldispersion sowie eine hinreichende optimale kohäsive Kraft im Trennlack, um eine geforderte Peelkraft zu erreichen.

Durch die besonders guten Siegeleigenschaften der Heißsiegeldispersion kann ein deutlich geringeres Auftragsgewicht gewählt werden. Dadurch ist wiederum eine optimale, erhöhte Effizienz auf der Beschichtungsanlage erreichbar.

Weitere Ausführungsformen der Erfindung ergeben sich aus den Unteransprüchen.

Nachfolgend wird die Erfindung anhand von Ausführungsbeispielen beschrieben, die anhand der Abbildungen näher erläutert werden. Hierbei zeigen
- Fig. 1: eine schematische Darstellung einer Ausführungsform der Erfindung; und
- Fig. 2: eine schematische Darstellung der Ausführungsform der Erfindung gemäß Fig. 1, wobei das erfindungsgemäße Verpackungsmaterial mit einem Gegenpart zunächst verbunden und sodann von diesem wieder getrennt wird.

In der nachfolgenden Beschreibung werden für gleiche und gleich wirkende Teile dieselben Bezugsziffern verwendet.

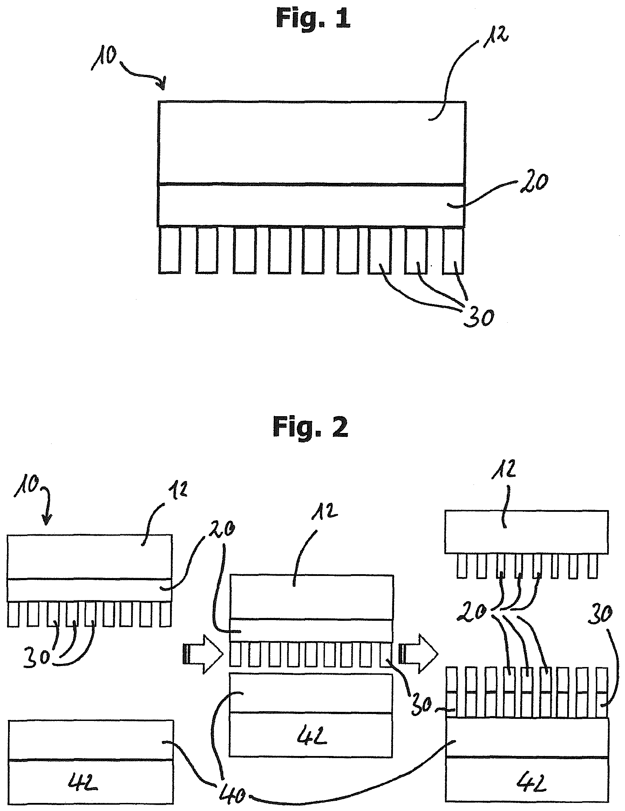

Fig. 1 zeigt eine Ausführungsform eines erfindungsgemäßen Verpackungsmaterials 10, das aus einem Verpackungsgrundmaterial 12 aus plasmabehandeltem Papier mit einem Flächengewicht von 60 g/m² besteht, auf dessen, wie in Fig. 1 dargestellt, untere Oberfläche ein Trennlack 20 auf Polyamidbasis mit einer Auftragsmenge von 1,5 g/m² vollflächig aufgebracht ist. Auf diesen Trennlack 20 ist wiederum ein Siegelmedium 30 gitterförmig aufgebracht, wobei das Auftragsgewichte Siegelmediums 3 g/m² beträgt.

In Fig. 2 ist eine schematische Darstellung der Ausführungsform der Erfindung gemäß Fig. 1 gezeigt, wobei das erfindungsgemäße Verpackungsmaterial 10 mit einem Gegenpart 40 zunächst verbunden und sodann von diesem wieder getrennt wird. Zu diesem Zweck wird das erfindungsgemäße Verpackungsmaterial 10 zunächst so mit dem Gegenpart 40 zusammengeführt, dass das Siegelmedium 30, das über den Trennlack 20 mit dem Verpackungsgrundmaterial 12 verbunden ist, an den Gegenpart 40, in diesem Fall Polyethylen, herangeführt und mit diesem versiegelt wird. Der Gegenpart 40 seinerseits ist auf einem Trägermaterial 42 angeordnet. Als Siegelmedium 30 wird erfindungsgemäß Ethylen-Vinylacetat-Copolymer verwendet, das mit einer optimalen Siegelkraft gegen Polyethylen siegelt. Beim Trennen des erfindungsgemäßen Verpackungsmaterial 10 von dem Gegenpart 40 verbleibt das Siegelmedium 30 an diesem Gegenpart 40. Mit dem Siegelmedium 30 verbleibt ferner ein Teil des Trennlacks 20 an dem Gegenpart 40, wobei der Trennlack 20 beim Trennen des erfindungsgemäßen Verpackungsmaterials 10 von dem Gegenpart 40 bricht und in den Bereichen, in welchen bei einem intakten Verpackungsmaterial 10, vor einem Verbinden des Verpackungsmaterials 10 mit dem Gegenpart 40, kein Siegelmedium 30 auf dem Trennlack 20 angeordnet war, an dem Verpackungsgrundmaterial 12 verbleibt. Somit stünde sich bei einem Versuch eines erneuten Verbindens des verbliebenen Verpackungsgrundmaterials 12 mit dem Trennlack eine ebenfalls aus Trennlack bestehende Schicht an dem Gegenpart 40 gegenüber, so dass eine erneute Verbindung des Verpackungsgrundmaterials 12 mit dem Gegenpart 40 nicht möglich ist.

Gemäß einem weiteren Ausführungsbeispiel der Erfindung wird eine plasmabehandelte Papierbahn 12 mit einem Flächengewicht von 80 g/m² vollflächig mit einem Trennlack 20 auf Polyamidbasis beschichtet. Der Trennlack 20 wird mit einem Flächengewicht von1,6 g/m² aufgetragen. Auf diesen Trennlack 20 wird wiederum eine wässrige Heißsiegeldispersion 30 mit einem Flächengewicht von 3,1 g/m² gitterförmig aufgetragen.

An dieser Stelle sei darauf hingewiesen, dass alle oben beschriebenen Teile für sich alleine gesehen und in jeder Kombination, insbesondere die in den Zeichnungen dargestellten Details, als erfindungswesentlich beansprucht werden. Abänderungen hiervon sind dem Fachmann geläufig.

### Bezugszeichenliste

- 10: Verpackungsmaterial
- 12: Verpackungsgrundmaterial
- 15: Papieraußenseite
- 20: Trennlack
- 30: Siegelmedium
- 40: Gegenpart
- 42: Trägermaterial des Gegenparts

## Patentansprüche

1. Verpackungsmaterial (10) aus Papier oder mit einer Papieraußenseite, insbesondere für medizinische Zwecke,
**dadurch gekennzeichnet, dass**
die Papieraußenseite (15), insbesondere vollflächig, mit einem Trennlack (20) und der Trennlack (20) musterförmig, nämlich gitterförmig, mit einem Siegelmedium (30) beschichtet ist, wobei der Trennlack (20) ein Trennlack (20) auf Polyamidbasis ist.

2. Verpackungsmaterial nach einem der vorhergehenden Ansprüche,
**dadurch gekennzeichnet, dass**
das Siegelmedium (30) aus einer Gruppe ausgewählt ist, die gegen Polyolefin(e), insbesondere Polyethylen oder Polypropylen, und/oder gegen Polyester, insbesondere Polyethylenterephtalat, und/oder gegen Papier und/oder gegen Polystyrol oder Mischungen vorgenannter Substanzen siegelt und Polyolefin(e), insbesondere Polyethylen oder Polypropylen, Polyester, insbesondere Polyethylenterephtalat, oder Mischungen vorgenannter Substanzen sowie Ethylen-Vinylacetat-Copolymer umfasst.

3. Verpackungsmaterial nach einem der vorhergehenden Ansprüche,
**dadurch gekennzeichnet, dass**
das Siegelmedium (30) in Form einer Dispersion auf den Trennlack (20) aufgebracht ist.

4. Verpackungsmaterial nach Anspruch 3,
**dadurch gekennzeichnet, dass**
die Dispersion eine Mischung von Wasser und Ethylen-Vinylacetat-Copolymer sowie gegebenenfalls einem Anteil eines Alkohols, insbesondere Ethanol, Propanol, Isopropanol oder Butanol, im Bereich von 0,01 Gew.-% bis 8 Gew.-%, bevorzugt im Bereich von 0,1 Gew.-% bis 6,5 Gew.-% und besonders bevorzugt im Bereich von 3,5 Gew.-% bis 5 Gew.-% und/oder gegebenenfalls einem weiteren Siegelmedium ist.

5. Verpackungsmaterial nach Anspruch 3,
**dadurch gekennzeichnet , dass**
die Dispersion außer Wasser kein weiteres, insbesondere kein weiteres organisches, Lösungsmittel umfasst und insbesondere eine Heißsiegeldispersion ist.

6. Verpackungsmaterial nach einem der vorhergehenden Ansprüche,
**dadurch gekennzeichnet, dass**
das Siegelmedium (30) mit einem Auftragsgewicht im Bereich von 0,5 g/m² bis 6 g/m², vorzugsweise im Bereich von 1,5 g/m² bis 5 g/m² und besonders bevorzugt im Bereich von 2 g/m² bis 4 g/m² auf den Trennlack (20) aufgebracht ist.

7. Verpackungsmaterial nach einem der vorhergehenden Ansprüche,
**dadurch gekennzeichnet, dass**
das Verpackungsmaterial (10) gasdurchlässig ist.

8. Verpackungsmaterial nach einem der vorhergehenden Ansprüche,
**dadurch gekennzeichnet, dass**
das Verpackungsmaterial (10) ein medizinisches Papier ist.

9. Verfahren zum Herstellen eines Verpackungsmaterials (10) nach einem der vorhergehenden Ansprüche,
**gekennzeichnet durch**
folgende Schritte:
- zur Verfügungstellen eines Verpackungsgrundmaterials (12) aus Papier oder mit einer Papieraußenseite (15);
- insbesondere vollflächiges, Beschichten der Papieraußenseite (15) mit einem Trennlack (20); und
- zumindest abschnittsweises, gitterförmiges, Aufbringen eines Siegelmediums (30) auf den Trennlack (20).

10. Verwendung eines Verpackungsmaterials (10) nach einem der vorhergehenden Ansprüche 1 bis 8 zum Herstellen eines, insbesondere medizinischen, Behälters.

## Claims

1. Packaging material (10) made of paper or with a paper outer side, in particular for medical purposes,
**characterized in that**
the paper outer side (15) is coated, in particular over the entire surface, with a release lacquer (20) and the release lacquer (20) is coated in a patterned manner, namely in a grid pattern, with a sealing medium (30), wherein the release lacquer (20) is a polyamide-based release lacquer (20).

2. Packaging material according to one of the preceding claims,
**characterized in that**
the sealing medium (30) is selected from a group that is impermeable to polyolefin(s), in particular polyethylene or polypropylene, and/or against polyester, in particular polyethylene terephthalate, and/or against paper and/or against polystyrene or mixtures of the aforementioned substances, and comprises polyolefin(s), in particular polyethylene or polypropylene, polyester, in particular polyethylene terephthalate, or mixtures of the aforementioned substances, as well as ethylene-vinyl acetate copolymer.

3. Packaging material according to one of the preceding claims,
**characterized in that**
the sealing medium (30) is applied to the release lacquer (20) in the form of a dispersion.

4. Packaging material according to claim 3,
**characterized in that**
the dispersion is a mixture of water and ethylene-vinyl acetate copolymer and, optionally, a proportion of an alcohol, in particular ethanol, propanol, isopropanol or butanol, in the range of 0.01 wt.% to 8 wt.%, preferably in the range of 0.1 wt.% to 6.5 wt.% and particularly preferably in the range of 3.5 wt.% to 5 wt.%, and/or optionally a further sealing medium.

5. Packaging material according to claim 3,
**characterized in that**
the dispersion does not contain any solvent other than water, in particular no other organic solvent, and is in particular a heat-sealing dispersion.

6. Packaging material according to one of the preceding claims,
**characterized in that**
the sealing medium (30) is applied to the release lacquer (20) with an application weight in the range of 0.5 g/m² to 6 g/m², preferably in the range of 1.5 g/m² to 5 g/m² and particularly preferably in the range of 2 g/m² to 4 g/m².

7. Packaging material according to one of the preceding claims,
**characterized in that**
the packaging material (10) is gas-permeable.

8. Packaging material according to one of the preceding claims,
**characterized in that**
the packaging material (10) is a medical paper.

9. Method for producing a packaging material (10) according to one of the preceding claims,
**characterized by**
the following steps:
- providing a packaging base material (12) made of paper or having a paper outer side (15);
- in particular, coating the paper outer side (15) over its entire surface with a release lacquer (20);
- and applying a sealing medium (30) to the release lacquer (20) at least in sections in a grid pattern.

10. Use of a packaging material (10) according to one of the preceding claims 1 to 8 for manufacturing a container, in particular a medical container.

## Revendications

1. Matériau d'emballage (10) en papier ou avec une face extérieure en papier, en particulier pour usages médicaux, **caractérisé en ce que** la face extérieure en papier (15) est enduite, en particulier sur toute sa surface, d'un vernis de séparation (20), et le vernis de séparation (20) est enduit selon un motif, à savoir selon un quadrillage, avec un agent de scellement (30), le vernis de séparation (20) étant un vernis de séparation (20) à base de polyamide.

2. Matériau d'emballage selon l'une des revendications précédentes, **caractérisé en ce que** l'agent de scellement (30) est choisi dans un groupe produisant un scellement contre des polyoléfines, en particulier du polyéthylène ou du polypropylène, et/ou des polyesters, en particulier du téréphtalate de polyéthylène, et/ou du papier et/ou du polystyrène ou des mélanges des substances susmentionnées et contient une ou des polyoléfines, en particulier du polyéthylène ou du polypropylène, un ou des polyesters, en particulier du téréphtalate de polyéthylène, ou des mélanges des substances susmentionnées ainsi qu'un copolymère d'éthylène et d'acétate de vinyle.

3. Matériau d'emballage selon l'une des revendications précédentes, **caractérisé en ce que** l'agent de scellement (30) est appliqué en dispersion sur le vernis de séparation (20).

4. Matériau d'emballage selon la revendication 3, **caractérisé en ce que** la dispersion est un mélange d'eau et de copolymère d'éthylène et d'acétate de vinyle avec éventuellement une part d'un alcool, en particulier, d'éthanol, de propanol, d'isopropanol ou de butanol, comprise entre 0,01 % en poids et 8 % en poids, de préférence entre 0,1 % en poids et 6,5 % en poids et en particulier entre 3,5 % en poids et 5 % en poids et/ou éventuellement d'un autre agent de scellement.

5. Matériau d'emballage selon la revendication 3, **caractérisé en ce que** la dispersion ne contient pas d'autre solvant que de l'eau, notamment pas d'autre solvant organique, et est en particulier une dispersion d'agent de scellement à chaud.

6. Matériau d'emballage selon l'une des revendications précédentes, **caractérisé en ce que** l'agent de scellement (30) est appliqué sur le vernis de séparation (20) à raison de 0,5 g/m² à 6 g/m², de préférence de 1,5 g/m² à 5 g/m² et en particulier de 2 g/m² à 4 g/m².

7. Matériau d'emballage selon l'une des revendications précédentes, **caractérisé en ce que** le matériau d'emballage (10) est perméable aux gaz.

8. Matériau d'emballage selon l'une des revendications précédentes, **caractérisé en ce que** le matériau d'emballage (10) est un papier pour usages médicaux.

9. Procédé pour la fabrication d'un matériau d'emballage (10) selon l'une des revendications précédentes, **caractérisé en ce qu'**il comprend les étapes suivantes :
- fourniture d'un matériau d'emballage de base (12) en papier ou avec une face extérieure en papier (15) ;
- enduction de la face extérieure en papier (15), en particulier sur toute la surface, avec un vernis de séparation (20) et
- application au moins par sections, en quadrillage, d'un agent de scellement (30) sur le vernis de séparation (20).

10. Utilisation d'un matériau d'emballage (10) selon l'une des revendications 1 à 8 pour la fabrication d'un récipient, en particulier pour usages médicaux.
